(19) Europäisches Patentamt
European Patent Office
Office européen des brevets

(11) EP 0 915 915 B1

# (12) EUROPÄISCHE PATENTSCHRIFT

(45) Veröffentlichungstag und Bekanntmachung des Hinweises auf die Patenterteilung:
**31.10.2001 Patentblatt 2001/44**

(21) Anmeldenummer: **97935551.8**

(22) Anmeldetag: **29.07.1997**

(51) Int Cl.⁷: **C08F 8/00**, A61K 7/06

(86) Internationale Anmeldenummer:
**PCT/EP97/04122**

(87) Internationale Veröffentlichungsnummer:
**WO 98/04596 (05.02.1998 Gazette 1998/05)**

## (54) WASSERLÖSLICHE POLYMERISATE UND DEREN VERWENDUNG IN KOSMETISCHEN FORMULIERUNGEN

WATER SOLUBLE POLYMERS AND THEIR USE IN COSMETIC FORMULATIONS

POLYMERES SOLUBLES DANS L'EAU ET LEUR UTILISATION DANS DES FORMULATIONS COSMETIQUES

(84) Benannte Vertragsstaaten:
**AT BE CH DE DK ES FR GB IT LI NL SE**

(30) Priorität: **31.07.1996 DE 19630977**

(43) Veröffentlichungstag der Anmeldung:
**19.05.1999 Patentblatt 1999/20**

(73) Patentinhaber: **BASF AKTIENGESELLSCHAFT
67056 Ludwigshafen (DE)**

(72) Erfinder:
- **NIESSNER, Manfred
D-67105 Schifferstadt (DE)**
- **RÜBENACKER, Martin
D-67122 Altrip (DE)**
- **NILZ, Claudia
D-67127 Rödersheim-Gronau (DE)**
- **HÖSSEL, Peter
D-67105 Schifferstadt (DE)**

(74) Vertreter: **Kinzebach, Werner, Dr. et al
Patent Attorneys,
Reitstötter, Kinzebach & Partner,
Sternwartstrasse 4
81679 München (DE)**

(56) Entgegenhaltungen:
**EP-A- 0 580 078**
**DE-A- 1 620 977**
**DE-A- 4 413 720**
**US-A- 2 484 423**

- **PATENT ABSTRACTS OF JAPAN vol. 095, no. 006, 31.Juli 1995 & JP 07 082320 A (SHOWA DENKO KK), 28.März 1995,**
- **PATENT ABSTRACTS OF JAPAN vol. 013, no. 515 (C-655), 17.November 1989 & JP 01 207311 A (AGENCY OF IND. SCIENCE & TECHNOL.), 21.August 1989,**

EP 0 915 915 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft wasserlösliche Polymerisate, Verfahren zu deren Herstellung und deren Verwendung in kosmetischen Mitteln.

Polymere finden in Kosmetik und Medizin vielfache Anwendung. In Seifen, Cremes und Lotionen beispielsweise dienen sie in der Regel als Formulierungshilfsmittel, z. B. als Verdicker, Schaumstabilisator oder Wasserabsorbens oder auch dazu, die Reizwirkung anderer Inhaltsstoffe abzumildern oder die dermale Applikation von Wirkstoffen zu verbessern. Ihre Aufgabe in der Haarkosmetik dagegen besteht darin, die Eigenschaften des Haares zu beeinflussen. So werden Conditioner dazu eingesetzt, um die Trocken- und Naßkämmbarkeit, Gefühl, Glanz und Erscheinungsform zu verbessern sowie dem Haar antistatische Eigenschaften zu verleihen. Weiterhin können sie verfestigend wirken, indem sie auf dem Haar hydrophobe Filme ausbilden.

Bevorzugt werden wasserlösliche Polymere mit polaren, häufig kationischen Funktionalitäten eingesetzt, die eine größere Affinität zur negativ geladenen Oberfläche des Haares aufweisen. Die Struktur und Wirkungsweise verschiedener Haarbehandlungspolymere sind in Cosmetics & Toiletries 103 (1988) 23 beschrieben. Handelsübliche Conditionerpolymere sind z.B. kationische Hydroxyethylcellulose, kationische Polymere auf der Basis von N-Vinylpyrrolidon, Acrylamid und Diallyldimethylammoniumchlorid oder Silikone.

In der US 4,713,236 sind Haarconditioner auf Basis von Vinylamin-einheiten enthaltenden Polymeren beschrieben. Genannt sind dabei insbesondere Polyvinylamin sowie dessen Salze, $\alpha$-substituierte Polyvinylamine wie z.B. Poly-($\alpha$-aminoacrylsäure) oder auch Copolymere, die neben Vinylamin Comonomere, wie Vinylalkohol, Acrylsäure, Acrylamid, Maleinsäureanhydrid, Vinylsulfonat und 2-Acrylamido-2-methylpropansulfonsäure einpolymerisiert enthalten.

Aus der US 5,478,553 ist die Anwendung von Homo- und Copolymeren von N-Vinylformamid als Haarfestiger insbesondere in wasserhaltigen Gelen bekannt. Als mögliche Comonomere werden dabei Styrol, Acryl- und Methacrylsäure, sowie deren Ester und Amide, Vinylester und Vinylether aufgeführt. In der WO 96/03969 ist darüber hinaus die Verwendung derartiger Polymere als Conditioner für Shampoos beschrieben.

In der JP 06 122 725 wird u.a. die Anwendung von pulverförmigen N-Vinylformamidhomo- und -copolymeren im Kosmetikbereich erwähnt.

Hydrolysierte Oligomere von N-Vinylformamid, die als Bestandteil von Körperpflegemittel eingesetzt werden können, sind aus der US 5,373,076 bekannt.

Aus der EP 510 246 sowie der japanischen Schrift J0 3223-304 sind vernetzte, vorwiegend anionische Homo- und Copolymerisate von N-Vinylcarbonsäureamiden bekannt, die als Wasserabsorber und Verdicker im Kosmetikbereich eingesetzt werden. Auch Copolymerisate aus Acrylamid und Methacrylsäure, die als weitere Monomere auch N-Vinylcarbonsäureamide enthalten können, finden gemäß DE 34 27 220 ebenfalls Anwendung als Verdicker.

In der EP 452 758 wird der Einsatz von wasserlöslichen Polymeren, die u.a. N-Vinylcarbonsäureamideinheiten enthalten, als Bestandteil von Gelen für dermale kosmetische und medizinische Anwendungen beschrieben. Gemäß DE 38 17 425 können auch vernetzte Hydrogele dafür verwendet werden.

Die aus dem Stand der Technik bekannten Conditioner-Polymere sind jedoch noch nicht völlig zufriedenstellend. Es besteht daher ein Bedarf an zusätzlichen Conditioner-Polymeren mit verbesserten Eigenschaften.

Auch aus anderen technischen Bereichen sind verschieden aufgebaute N-Vinylamid-Polymerisate bekannt.

So sind beispielsweise Polymerisate, die N-Vinyl-N-alkylcarbonsäureamideinheiten einpolymerisiert enthalten, in der DE-OS 28 29 652 beschrieben. Poly-N-vinyl-N-methylformamid wird in der US 3 558 581, Poly-N-vinyl-N-methylacetamid in der GB 1 082 016 genannt.

Homopolymere des N-Vinylformamids sind aus der US 4 421 602 und der EP-B-0071050 bekannt. Diese Polymeren dienen als Vorstufe für Vinylamineinheiten einpolymerisiert enthaltende Polymeren. So werden beispielsweise Copolymere, die 90 bis 10 mol-% N-Vinylformamid- und 10 bis 90 mol-% Vinylamineinheiten einpolymerisiert enthalten, beschrieben. Diese Polymeren erhält man durch radikalische Polymerisation von N-Vinylformamid und nachfolgende hydrolytische Abspaltung eines Teils der Formylreste mit Hilfe von Säuren oder Basen. Diese Polymere finden als Hilfsmittel bei der Papierherstellung oder als Flockungsmittel für Schlämme Verwendung.

In der EP 0 216 387 werden Copolymere beschrieben, die neben N-Vinylformamid auch andere ethylenisch ungesättigte Monomere wie Vinylacetat, Vinylpropionat, $C_1$- bis $C_4$-Alkylvinylether, Ester, Nitrile und Amide von Acryl- und Methacrylsäure sowie N-Vinylpyrrolidon enthalten. Durch partielle oder vollständige Hydrolyse dieser Copolymeren gelangt man ebenfalls zu Copolymerisaten, die Vinylamineinheiten einpolymerisiert enthalten. Aus der EP 0 251 182 sind Copolymerisate bekannt, die neben N-Vinylformamid- und Vinylamin auch Acrylnitrileinheiten sowie gegebenenfalls geringe Anteile an Acrylamid- und Acrylsäureeinheiten enthalten. Auch diese Copolymerisate finden Anwendung in der Papierindustrie.

Die Herstellung dieser bekannten Polymeren erfolgt dabei in der Regel durch radikalische Homo- und Copolymerisation der entsprechenden Monomeren. Aufgrund der schlechten Verfügbarkeit speziell substituierter N-Vinylcarbonsäureamide oder ihrer schlechten Polymerisierbarkeit, bietet es sich an, durch Reaktion von primären und sekundären, Vinylamineinheiten einpolymerisiert enthaltenden Polymerisaten mit Carbonsäurederivaten zu N-Vinylcarbonsäureamidfunktionen zu gelangen. In Macromol. Chem. Phys. 195 (1994) 679 ist die Umsetzung von Polyvinylamin mit aliphatischen Carbonsäurechloriden beschrieben. Die Reaktion mit aromatischen Sulfonsäurechloriden zu den entsprechenden Sulfonamiden ist aus der US 4,403,072 und der US 4,275,002 bekannt. Die Bildung von Amidfunktionen ausgehend von Vinylamineinheiten einpolymerisiert enthaltenden Polymerisaten durch Umsetzung mit Carbonsäureestern ist dagegen nicht beschrieben.

Die Umsetzung von Polyvinylamin bzw. dessen Hydrochlorid mit verschiedenen Zuckerlactonen, z. B. Glucolacton ist in Macromol. Chem. 184 (1983) 1441 beschrieben. Diese Reaktionen werden generell in nicht wäßrigen, polaren Lösungsmitteln durchgeführt, wozu in wäßriger Lösung synthetisierte Polymere erst in aufwendiger Weise isoliert werden müssen.

Badesso et al. beschreiben in Adv. Chem. Ser. (1996), 248, 490-504 die Herstellung von Polyvinylamin durch Hydrolyse von Poly(N-ethenylformamid). Außerdem wird die Herstellung einzelner Polyvinylamid-Derivate beschrieben. Unter anderem wird ein Reaktionsprodukt von Polyvinylamin und γ-Butyrolacton beschrieben, wobei 24 mol-% der Amineinheiten mit einem Rest der Formel

$$-C(O)-CH_2-CH_2-CH_2-OH$$

substituiert sind. Als Lösungsmittel wird dabei Methanol verwendet. Als mögliche Anwendungsgebiete für das Butyrolacton-Derivat werden die Abwasserbehandlung und die Papierherstellung genannt. Eine möglicherweise vorteilhafte Anwendbarkeit des Derivats in kosmetischen Mitteln wird nicht erwähnt.

Aufgabe der vorliegenden Erfindung ist somit die Bereitstellung neuartiger N-Vinylamid-Polymerisate mit verbesserten kosmetischen Eigenschaften. Insbesondere sollten Polymerisate bereitgestellt werden, welche die Naßkämmbarkeit von Kopfhaar verbessern.

Diese Aufgabe wird überraschenderweise gelöst durch Verwendung von Polymerisaten, die als wesentliche Strukturelemente hydroxysubstituierte N-Vinylcarbonsäureamid-Einheiten umfassen.

Soweit keine anderen Angaben gemacht werden, gelten bei der nun folgenden konkreten Beschreibung der Erfindung die folgenden Definitionen.

Erfindungsgemäß verwendbare Alkylreste umfassen geradkettige oder verzweigte, gesättigte Kohlenstoffketten mit 1 bis 20 Kohlenstoffatomen. Beispielsweise können folgende Reste genannt werden: Alkylreste mit 1 bis 12 Kohlenstoffatomen, wie die $C_1$-$C_6$-Alkylreste Methyl, Ethyl, n-Propyl, i-Propyl, n-Butyl, sec.-Butyl, i-Butyl, t-Butyl, n-Pentyl, sec.-Pentyl, i-Pentyl, n-Hexyl, 1-, 2- oder 3-Methyl-pentyl; und längerkettige Reste, wie unverzweigtes Heptyl, Octyl, Nonyl, Decyl, Undecyl, Lauryl und die ein- oder mehrfach verzweigten Analoga davon; sowie Alkylreste mit mehr als 12 Kohlenstoffatomen, wie unverzweigtes Tridecyl, Myristyl, Pentadecyl, Palmityl, Heptadecyl, Stearyl, Nonadecyl und Eicosyl sowie die ein- oder mehrfach verzweigten Analoga davon.

Erfindungsgemäß verwendbare Cycloalkylreste umfassen insbesondere $C_3$-$C_{12}$-Cycloalkylreste, wie z.B. Cyclopropyl, Cyclobutyl, Cyclopentyl, Cyclohexyl, Cycloheptyl, Cyclooctyl, Cyclopropylmethyl, Cyclopropylethyl, Cyclopropylpropyl, Cyclobutylmethyl, Cyclobutylethyl, Cyclopentylethyl, Cyclopentylpropyl, -butyl, -pentyl und -hexyl.

Beispiele für erfindungsgemäß brauchbare Arylreste sind Phenyl und Naphthyl, insbesondere Phenyl.

Beispiele für erfindungsgemäß brauchbare Aralkylreste sind Aryl-$C_1$-$C_{10}$-alkyl- und insbesondere Aryl-$C_1$-$C_6$-alkyl-Reste, wobei Aryl- und Alkylteil wie oben definiert sind.

Die Summe aller Prozentangaben (mol-%, Gew.-%) zur Beschreibung einer Zusammensetzung beträgt jeweils 100.

Ein erster Gegenstand der Erfindung betrifft zur Verwendung in kosmetischen Mitteln besonders geeignete Polymerisate, die dadurch gekennzeichnet sind, daß sie als wesentliche Strukturelemente Einheiten der Formel I und/oder II

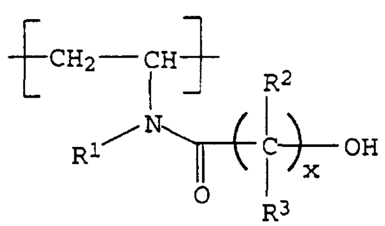

(I)

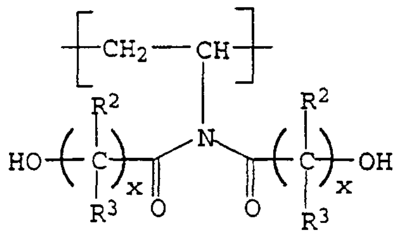 (II)

umfassen, worin

$R^1$ für H, Alkyl, vorzugsweise $C_1$-$C_{12}$-Alkyl, Cycloalkyl, vorzugsweise $C_3$-$C_{12}$-Cycloalkyl, Aryl, vorzugsweise Phenyl, oder Aralkyl, vorzugsweise Phenyl-$C_1$-$C_6$-alkyl, steht,

$R^2$ und $R^3$ unabhängig voneinander wie $R^1$ definiert sind, wobei jedoch Alkyl vorzugsweise eine $C_1$-$C_{20}$-Alkylgruppe ist, oder ausgewählt sind unter den entsprechenden ein- oder mehrfach substituierten Alkyl-, Cycloalkyl-, Aryl- oder Aralkylresten, wobei die Substituenten ausgewählt sind unter OH, O-Alkyl, O-Aryl, SH, S-Alkyl, S-Aryl, $NH_2$, NH-Alkyl, NH-Aryl, $N(Alkyl)_2$, gegebenenfalls in protonierter Form, $N(Alkyl)_3^+Z$, worin Z für den Rest einer anorganischen oder organischen Säure steht, COOH, COO-Alkyl, $CONH_2$, CONH-Alkyl, $CON(Alkyl)_2$, CN und $SO_3H$; und

die Indices x jeweils unabhängig voneinander für eine ganze Zahl von 1 bis 20, insbesondere 1 bis 10, vorzugsweise 1 bis 6, stehen;

sowie die entsprechenden Säureadditionssalze dieser Polymerisate.

Zur Verwendung in kosmetischen Mitteln sind auch solche Polymere geeignet, worin $R^2$ und $R^3$ unabhängig voneinander für OH stehen. Bevorzugte Substituenten der Reste $R^2$ und $R^3$ sind -OH, -O-$C_1$-$C_6$-Alkyl, -0-Phenyl, -SH, -S-$C_1$-$C_6$-Alkyl, -S-Phenyl, -$NH_2$, -NH-$C_1$-$C_6$-Alkyl, -NH-Phenyl, -N($C_1$-$C_6$-Alkyl)$_2$, —N($C_1$-$C_6$-Alkyl)$_3^+$$Z^-$, -COOH, -COO-$C_1$-$C_6$-Alkyl, -$CONH_2$, -CONH-$C_1$-$C_6$-Alkyl, -CON($C_1$-$C_6$-Alkyl)$_2$, -CN und -$SO_3H$.

Bevorzugte Gegenionen $Z^-$ sind, $Cl^-$, $Br^-$, $H_2PO_4^-$, $SO_4^{2-}$, $NO_3^-$, $HCOO^-$, Phenyl-$SO_3^-$.

Gemäß einer bevorzugten Ausführungsform der Erfindung werden neuartige Polymerisate bereitgestellt, die

a) 0,1 bis 100 mol-%, vorzugsweise 25 bis 100 mol-%, wie z. B. 30 bis 90, 30 bis 80, 30 bis 70 oder 30 bis 60 mol-%, Einheiten obiger allgemeiner Formeln I und/oder II

b) 0 bis 99,9 mol-%, vorzugsweise 0 bis 75 mol-%, wie z. B. 10 bis 70, 20 bis 70, 30 bis 70 oder 40 bis 70 mol-%, Vinylamineinheiten der allgemeinen Formel III

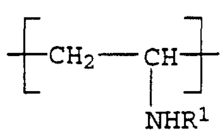 (III)

worin $R^1$ die oben angegebenen Bedeutungen besitzt,

c) 0 bis 99,9 mol-%, vorzugsweise 0 bis 60 mol-%, wie z. B. 0 bis 50, 0 bis 40, oder 20 bis 40 mol-%, von a) und b) verschiedener monoethylenisch ungesättigter Monomereinheiten, vorzugsweise ausgewählt unter N-Vinylcarbonsäureamiden wie z.B. N-Vinyl-$C_1$-$C_6$-carbonsäureamiden, N-Vinylpyrrolidon, N-Vinylcaprolactam, N-Vinylharnstoff, N-Vinylimidazolen, N-Vinylimidazolinen, Vinylalkohol, Vinylformiat, Vinylacetat, Vinylpropionat, Vinylbutyrat, $C_1$-$C_6$-Vinylether, monoethylenisch ungesättigten $C_3$-$C_8$-Carbonsäuren und -Dicarbonsäuren sowie deren Ester, Amiden, Anhydriden und Nitrilen; und

d) 0 bis 5 mol-%, wie z. B. 0 bis 3, 0 bis 2 oder 0 bis 1 mol-%, mindestens diethylenisch ungesättigter Monomereinheiten, vorzugsweise Methylenbisacrylamid, Glykoldiacrylat, Glycerintriacrylat, Glykoldimethacrylat, Glycerintrimethacrylat, Divinylbenzol, Divinyldioxan, Pentaerythrittriallylether, Pentaallylsucrose, Divinylharnstoff und Divinylethylenharnstoff;

umfassen.

Besonders bevorzugt werden Polymerisate aus

a) 1 bis 100 mol-%, insbesondere etwa 5 bis 100 mol-%, wie z. B. 5 bis 80, 10 bis 80, 25 bis 80, 25 bis 60, 30 bis 80 oder 30 bis 60 mol-%, Einheiten der Formel I und/oder II, worin $R^1$ für H steht; $R^2$ und $R^3$ unabhängig vonein-

ander ausgewählt sind unter H, $C_1$-$C_6$-Alkyl und Phenyl; und x für eine ganze Zahl von 1 bis 10 steht,

b) 0 bis 99 mol-%, insbesondere etwa 0 bis 95 mol-%, wie z. B. 5 bis 90, 10 bis 90, 20 bis 90, 30 bis 90 oder 30 bis 80 mol-%, Vinylamin-Einheiten der Formel III, worin $R^1$ für H steht, und

c) 0 bis 99 mol-%, insbesondere etwa 0 bis 95 mol-%, wie z. B. 0 bis 60, 0 bis 50, 0 bis 40 oder 20 bis 40 mol-%, wenigstens eines anderen ethylenisch ungesättigten Monomers, ausgewählt unter N-Vinylformamid, N-Vinylpyrrolidon, N-Vinylcaprolactam, N-Vinylharnstoff, Vinylalkohol, Vinylformiat, Vinylacetat, Vinylpropionat, $C_1$-$C_6$-Vinylether, Acrylsäure, Methacrylsäure sowie deren Ester, Amiden, Anhydriden und Nitrilen

in kosmetischen Mitteln eingesetzt.

Insbesondere bevorzugt verwendet man Polymerisate aus

a) 1 bis 100 mol-%, insbesondere etwa 5 bis 95 mol-%, wie z. B. 5 bis 80, 10 bis 80, 25 bis 80, 25 bis 60, 30 bis 80 oder 30 bis 60 mol-%, Einheiten der Formel I und/oder II, worin $R^1$ für H steht; eine der Reste $R^2$ und $R^3$ für H steht und der andere Rest für H oder Methyl steht; und x für eine ganze Zahl von 1 bis 6 steht; und

b) 0 bis 99 mol-%, insbesondere etwa 0 bis 95 mol-%, wie z. B. 5 bis 90, 10 bis 90, 20 bis 90, 30 bis 90 oder 30 bis 80 mol-%, Vinylamin-Einheiten; und

c) 0 bis 99 mol-%, insbesondere etwa 0 bis 95 mol-%, wie z. B. 0 bis 60, 0 bis 50, 0 bis 40 oder 20 bis 40 mol-%, N-Vinylformamid-Einheiten.

Gegenstand der vorliegenden Erfindung ist außerdem ein Verfahren zur Herstellung von Polymerisaten, die als wesentliche Strukturelemente Einheiten der Formel IV und/oder V

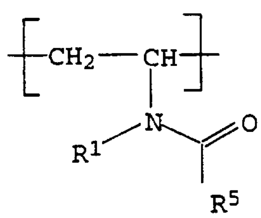 (IV) 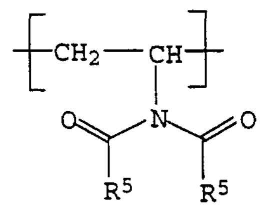 (V)

umfassen, worin

$R^1$ für Alkyl, Cycloalkyl, Aryl oder Aralkyl steht, und

die Reste $R^5$ unabhängig voneinander ausgewählt sind unter Alkyl, Cycloalkyl, Aryl und Aralkyl, gegebenenfalls ein- oder mehrfach substituiert mit OH, O-Alkyl, 0-Aryl, SH, S-Alkyl, S-Aryl, $NH_2$, NH-Alkyl, NH-Aryl, $N(Alkyl)_2$, gegebenenfalls in protonierter Form, $N(Alkyl)_3^+Z^-$, worin Z für den Rest einer anorganischen oder organischen Säure steht, COOH, COO-Alkyl, $CONH_2$, CONH-Alkyl, $CON(Alkyl)_2$, CN oder $SO_3H$; oder für einen Rest der Formel VI

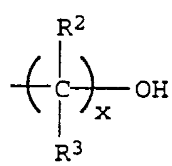 (VI)

stehen, worin $R^2$, $R^3$ und x die oben angegebenen Bedeutungen besitzen und $R^2$ und $R^3$ außerdem unabhängig voneinander für OH stehen können,

und der entsprechenden Säureadditonssalze dieser Polymerisate,
das dadurch gekennzeichnet ist, daß man ein Präpolymerisat, enthaltend Vinylamineinheiten der Formel III

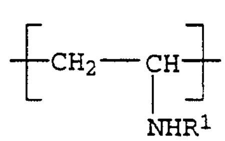 (III)

worin $R^1$ die oben angegebenen Bedeutungen besitzt, mit einer Verbindung der Formel VII

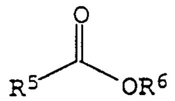

(VII)

worin

| | |
|---|---|
| $R^5$ | die oben angegebenen Bedeutungen besitzt und |
| $R^6$ | für Alkyl, Aralkyl oder Aryl steht, oder |
| $R^5$ und $R^6$ | zusammen für eine Gruppe der Formel VIa |

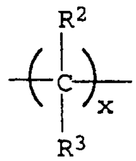

(VIa)

stehen, worin $R^2$, $R^3$ und x die oben angegebenen Bedeutungen besitzen,

umsetzt, und das Produkt gegebenenfalls in das entsprechende Säureadditionssalz überführt.

Insbesondere setzt man ein Präpolymerisat ein, das

b) 0,1 bis 100 mol-% Vinylamineinheiten der Formel III,
c) 0 bis 99,9 mol-% von a) verschiedener monoethylenisch ungesättigter Monomereinheiten und
d) 0 bis 5 mol-% mindestens diethylenisch ungesättigter Monomereinheiten umfaßt.

Die Komponenten b), c) und d) sind dabei wie oben angegeben definiert.

Unter Anwendung des erfindungsgemäßen Verfahrens sind somit nicht nur die neuartigen, durch die hydroxylierten N-Vinylcarbonsäureamid-Einheiten der Formeln I und II charakterisierten Polymerisate, sondern darüber hinaus auch alle anderen, durch Einheiten der allgemeinen Formeln IV und V charakterisierten Polymere herstellbar.

Gemäß einer ersten bevorzugten Variante des erfindungsgemäßen Verfahrens stellt man Polymerisate her, die durch Einheiten der allgemeinen Formeln I und/oder II gekennzeichnet sind. Hierzu setzt man ein Präpolymerisat gemäß obiger Definition mit Lactonen der Formel VIII,

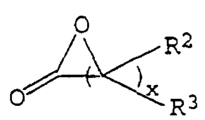

(VIII)

worin $R^2$ und $R^3$ unabhängig voneinander für H, OH, oder einen $C_1$-$C_{20}$-Alkyl-, $C_3$-$C_{12}$-Cycloalkyl-, Aryl- oder Aralkyl-Rest stehen, der gegebenenfalls substituiert ist durch eine funktionelle Gruppe wie OH, O-Alkyl, O-Aryl, SH, S-Alkyl, S-Aryl, $NH_2$, NH-Alkyl, NH-Aryl, $N(Alkyl)_2$ als freies Amin und/oder in protonierter Form, $N(Alkyl)_3^+Z^-$, COOH, COO-Alkyl, $CONH_2$,

CONH-Alkyl, $CON(Alkyl)_2$, CN oder $SO_3H$; und x eine ganze Zahl von 1 bis 20 bedeutet, um.

Geeignete Lactone sind dabei beispielsweise Acetolacton (x = 1), 2-Methylacetolacton, 2,2-Dimethylacetolacton, 2,2-Diphenylacetolacton, 2-Ethylacetolacton, 2,2-Diethylacetolacton, 2-Benzylacetolacton, β-Propiolacton (x = 2), 2-Methyl-β-propiolacton, 3-Methyl-β-propiolacton, 2,3-Dimethyl-β-propiolacton, 3,3-Dimethyl-β-propiolacton, 3-Phenyl-β-propiolacton, 3-Ethyl-β-propiolacton, 3-Benzyl-β-propiolacton, γ-Butyrolacton (x = 3), 4-Methyl-γ-butyrolacton, 3-Methyl-γ-butyrolacton, 4-Ethyl-γ-butyrolacton, 3,3-Dimethyl-γ-butyrolacton, 4-Phenyl-γ-butyrolacton, 4-Benzyl-γ-butyrolacton, δ-Valerolacton (x = 4), γ-Valerolacton, 2-Methyl-δ-valerolacton, 5-Phenyl-δ-valerolacton, 5-Benzyl-δ-valerolacton, 2-Ethyl-δ-valerolacton, 3-Hydroxy-3-methyl-δ-valerolacton, ε-Caprolacton (x = 5) sowie Exaltolid (x = 18) oder auch hydroxysubstituierte Lactone wie Milchsäurelacton oder Mandelsäurelacton. Vorzugsweise verwendet man β-Propiolacton, γ-Butyrolacton, γ-Valerolacton, δ-Valerolacton und ε-Caprolacton.

In Frage kommen auch Lactone von Aldonsäuren, z. B. Glycerinsäure, Threonsäure, Erythronsäure, Ribonsäure, Arabinonsäure, Xylonsäure, Lyxonsäure, Allonsäure, Atronsäure, Gluconsäure, Mannonsäure, Gulonsäure, Idonsäure, Galactonsäure und Talonsäure oder Aldonsäurelactone von Di- und Oligosacchariden wie Maltose oder Cellubiose.

Gemäß einer zweiten bevorzugten Variante des erfindungsgemäßen Verfahrens setzt man ein Präpolymerisat gemäß obiger Definition mit Carbonsäureestern der Formel (VII)

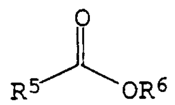

(VII)

um, worin
$R^5$ für einen $C_1$-$C_{20}$-Alkyl- $C_3$-$C_{12}$-Cycloalkyl-, Aryl- oder Aralkyl-Rest steht, der gegebenenfalls substituiert ist durch eine funktionelle Gruppe ausgewählt unter OH, O-Alkyl, 0-Aryl, SH, S-Alkyl, S-Aryl, $NH_2$, NH-Alkyl, NH-Aryl, $N(Alkyl)_2$ als freies Amin und/oder in protonierter Form, $N(Alkyl)_3^+Z^-$, COOH, COO-Alkyl,

$CONH_2$, CONH-Alkyl, $CON(Alkyl)_2$, CN und $SO_3H$; und $R^6$ für $C_1$-$C_6$-Alkyl, Aralkyl oder Aryl steht.

Bei dieser Verfahrensvariante setzt man bevorzugt Präpolymere ein, die als Einheiten

a) 5 bis 100 mol-% primäres Vinylamin und
b) 0 bis 95 mol-% anderen ethylenisch ungesättigten Monomeren aus der Gruppe N-Vinylformamid, N-Vinylpyrrolidon, N-Vinylcaprolactam, N-Vinylharnstoff, Vinylalkohol, Vinylformiat, Vinylacetat, Vinylpropionat, Acrylsäure und/oder Acrylnitril,

einpolymerisiert enthalten.

Als Beispiele für geeignete Ester der Formel VII können genannt werden Methyl-, Ethyl-, Propyl,- Isopropyl-, Benzyl-, Phenyl-, n-Butyl-, n-Pentyl- und n-Hexylester aliphatischer und aromatischer Monocarbonsäuren, wie z.B. Essigsäure, Propionsäure, Buttersäure, Isobuttersäure, n-Valeriansäure, Pivalinsäure, Capronsäure, Cyclopentylcarbonsäure, Cyclohexylcarbonsäure, Önanthsäure, Caprylsäure, Nonansäure, Caprinsäure, Laurinsäure, Myristinsäure, Palmitinsäure, Stearinsäure, Benzoesäure, 1-Naphtalincarbonsäure, 2-Naphtalincarbonsäure, Phenylessigsäure und β-Phenylpropionsäure oder Dicarbonsäuren, wie beispielsweise Oxalsäure, Malonsäure, Bernsteinsäure, Glutarsäure, Adipinsäure, Pimelinsäure und Korksäure. Diese Carbonsäuren können gegebenenfalls auch durch funktionelle Gruppen, wie OH, 0-Alkyl, 0-Aryl, Halogen, SH, S-Alkyl, S-Aryl, $NH_2$, NH-Alkyl, NH-Aryl, $N(Alkyl)_2$ als freies Amin und/ oder in protonierter Form, $N(Alkyl)_3^+Z^-$, COOH, COO-Alkyl, $CONH_2$, CONH-Alkyl, $CON(Alkyl)_2$, CN, $SO_3H$, substituiert sein. Beispiele substituierter Carbonsäuren sind Chloressigsäure, Bromessigsäure, Cyanessigsäure, α-Chlorpropionsäure, β-Chlorpropionsäure, α-Brompropionsäure, β-Brompropionsäure, Glykolsäure, Milchsäure, Äpfelsäure, Weinsäure, Mandelsäure, Salicylsäure, Mercaptoessigsäure, α-Mercaptopropionsäure, β-Mercaptopropionsäure, Glycin, N-Methylglycin, N,N-Dimethylglycin, Cholin, α-Alanin, β-Alanin, Valin, Leucin, Isoleucin, Serin, Threonin, Lysin, Phenylalanin, Thyrosin und Prolin. Geeignet sind auch Ester mehrwertiger Alkohole, wie beispielsweise Glykol oder Polyglykole, Glycerin, Mannit, Sorbit, Glukose, wobei eine oder mehrere Hydroxylgruppen mit Carbonsäuren verestert sein können. Vorzugsweise verwendet man Essigsäure-, Propionsäure-, Buttersäure-, Benzoesäure, Laurinsäure-, Palmitinsäure- und Stearinsäuremethylester oder Triglyceride höherer Fettsäuren, wie Laurinsäure, Palmitinsäure und Stearinsäure.

Reaktionen von Carbonsäureestern und Lactonen mit Aminen sind in der Literatur vielfach beschrieben. Da die zuvor genannten, Vinylamineinheiten einpolymerisiert enthaltenden Präpolymere vorwiegend wasserlöslich sind, ist es zweckmäßig, die Reaktion mit den Estern oder Lactonen der Formeln VII bzw. VIII in Gegenwart von Wasser durchzuführen. So kann die Umsetzung sowohl in wäßriger Lösung als auch in Mischungen von Wasser mit anderen inerten Lösungsmitteln erfolgen. Geeignete Lösungsmittel sind beispielsweise solche, in denen die Ester und Lactone löslich sind, wie Methanol, Ethanol, Isopropanol, n-Propanol, n-Butanol, sek.-Butanol, tert.-Butanol, Glykol, Dimethylethylenglykol, Tetrahydrofuran, Dioxan, Hexamethylphosphorsäuretriamid, Acetonitril oder Aceton, sowie Mischungen der genannten Lösungsmittel. Bevorzugt erfolgt die Umsetzung in wäßriger Lösung bei pH-Werten von 7 bis 12, vorzugsweise 9 bis 11. Es ist auch möglich, die Reaktion in gepufferter Lösung durchzuführen, wobei sich vor allem das Puffersystem primäres/sekundäres Phosphat eignet. Die Konzentration des Polymeren in der wäßrigen Lösung beträgt je nach Molekulargewicht 5 bis 60 Gew.-%, vorzugsweise 10 bis 30 Gew.-%, so daß die Lösung während der Reaktion gut rührbar ist.

Die Reaktionstemperatur beträgt 20 bis 200°C, vorzugsweise 40 bis 120°C und besonders bevorzugt 50 bis 100°C. Führt man die Reaktion bei Temperaturen oberhalb der Siedetemperatur des Esters bzw. Lactons oder des

inerten Lösungsmittels durch, wird die Reaktion unter Druck in einem entsprechenden Druckbehälter durchgeführt. Die Reaktionszeit beträgt 1 bis 20 Stunden, vorzugsweise 3 bis 10 Stunden. Die bei der Umsetzung der Aminfunktionen mit Estern freigesetzten Alkohole können gegebenenfalls im Verlauf der Reaktion destillativ entfernt und so dem Gleichgewicht entzogen werden.

Je nach angestrebtem Amidierungsgrad im Endprodukt fügt man pro umzusetzende Vinylamineinheit 1 bis 5, vorzugsweise 1 bis 2,5 Äquivalente Ester oder Lacton zu. Sekundäre Vinylamineinheiten ($R^1 \neq H$) reagieren dabei ausschließlich zu Strukturen der Formel I. Im Fall von primären Aminfunktionen entstehen, je nach eingesetzter Menge an Lacton, sowohl Strukturen I als auch II. Setzt man das Lacton im Unterschuß zu, so ist die Bildung von Struktur I bevorzugt.

Durch die Amidbildung wird die Anzahl basischer Gruppen und damit die kationische Ladungsdichte des Polymeren verringert, was durch Polyelektrolyttitration nachweisbar ist. Der Umsatz bezüglich des eingesetzten Lactons beträgt in der Regel mehr als 50 %, in den meisten Fällen mehr als 70 %. Nach Vollendung der Reaktion kann die Lösung durch Zusatz von Säuren oder Basen auf den gewünschten pH-Wert eingestellt werden. Es besteht aber auch die Möglichkeit, das Polymere durch Fällung in Alkoholen oder Aceton zu isolieren oder die Lösung dialytisch von niedermolekularen Bestandteilen zu befreien. Die Zusammensetzung kann durch Elementaranalyse sowie $^1H$-NMR- und IR-Spektroskopie bestimmt werden.

Die erhaltenen Polymerisate haben insbesondere Molekulargewichte von 1000 bis 10 Millionen, vorzugsweise 10 000 bis 5 Millionen, was K-Werten von etwa 5 bis 300 bzw. 10 bis 250 entspricht, gemessen an 1%-igen wäßrigen Lösungen bei pH 7 und 25°C nach H. Fikentscher, Cellulose-Chemie, Band 13, 58 bis 64 und 71 bis 74 (1932).

Um bei der Lagerung eine weitgehend farbstabile Polymerlösung zu erhalten, setzt man nach der Umsetzung mit den Estern oder Lactonen gegebenenfalls Antioxidantien, Reduktionsmittel oder Aldehydfänger zu. Antioxidantien, die meist als Radikalfänger oder UV-Stabilisatoren wirken, sind beispielsweise sekundäre aromatische Amine, Phenol, Alkylphenole, Thioether, Phosphite oder Mischungen von Verbindungen der genannten Stoffklassen. Geeignete sekundäre aromatische Amine sind z.B. 4,4'-Bis(phenylmethyl-)diphenylamin, 4,4'-Bis(tert. butyl)diphenylamin oder deren Gemische. Als Antioxidantien geeignete Alkylphenole sind z.B. 2,6-Dimethyl-4-tert. butylphenol, 2,4,6-Trimethylphenol, 2,4-Di-tert.-butyl-6-methylphenol oder deren Gemische. Als Thioether kommen in Frage Dialkyl-3,3'-thiodipropionat, Poly-2,3-dimethylphenyl-1,4-disulfid, Dibenzylsulfid und Dialkyldisulfide wie beispielsweise Dioctadecyldisulfid. Phosphite, die als Antioxidantien in Betracht kommen, sind z.B. Trisnonylphenylphosphit, Di-(2,4-di-tert. butylphenyl)-pentaerythritoldiphosphit und Diphenylen-decylphosphit.

Als Reduktionsmittel eignen sich beispielsweise Natriumborhydrid, Natriumcyanoborhydrid, Dithionite, wie Natrium-, Kalium- oder Zinkdithionit oder auch unterphosphorige Säure.

Aldehydfänger sind beispielsweise NH-Gruppen aufweisende Verbindungen, wie Harnstoff, Ethylenharnstoff, Melamin, Guanidin, Phenylguanidin oder Mischungen der genannten Verbindungen. Zu nennen sind auch Alkalimetallbisulfite wie Natrium- oder Kaliumbisulfit.

Antioxidantien, Reduktionsmittel und Aldehydfänger werden jeweils in Mengen von 0,01 bis 20 Gew.-%, vorzugsweise 0,1 bis 16 Gew.-%, bezogen auf die Polymerisate, eingesetzt.

Die als Ausgangsmaterial für die Durchführung des erfindungsgemäßen Verfahrens erforderlichen Präpolymerisate erhält man nach bekannten Verfahren, beispielsweise durch radikalisch initiierte Homo- und Copolymerisation von N-Vinylcarbonsäureamiden der Formel IX,

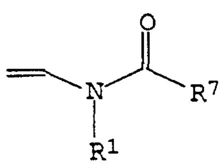

(IX)

worin $R^1$ die oben angegebenen Bedeutungen besitzt und $R^7$ für H oder $C_1$-$C_6$-Alkyl steht, und anschließender partieller oder vollständiger, hydrolytischer Abspaltung der Gruppierung

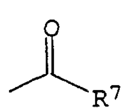

vorzugsweise mit Hilfe von Säuren, Basen oder Enzymen.

Die Hydrolyse wird vorzugsweise in Wasser unter Einwirkung von Säuren, Basen oder Enzymen durchgeführt, kann jedoch auch in Abwesenheit der genannten Hydrolysemittel erfolgen. In Abhängigkeit von den Reaktionsbedingungen bei der Hydrolyse, d.h. der Menge an Säure oder Base, bezogen auf das zu hydrolysierende Polymerisat sowie Reaktionszeit und -temperatur, erhält man verschiedene Hydrolysegrade. Die Hydrolyse wird soweit geführt, daß 0,1 bis 100 mol-%, bevorzugt 1 bis 99 mol% und ganz besonders bevorzugt 5 bis 95 mol-% der Carbonsäurereste hydrolytisch abgespalten werden.

Für die Hydrolyse geeignete Säuren sind beispielsweise Mineralsäuren, wie Halogenwasserstoff (gasförmig oder in wäßriger Lösung), Schwefelsäure, Salpetersäure, Phosphorsäure (ortho-, meta- oder Polyphosphorsäure) oder organische Säuren, z.B. $C_1$-$C_5$-Carbonsäuren, wie Ameisensäure, Essigsäure oder Propionsäure oder aliphatische und aromatische Sulfonsäuren, wie Methansulfonsäure, Benzolsulfonsäure oder Toluolsulfonsäure. Bei der Hydrolyse mit Säuren beträgt der pH-Wert 0 bis 5. Pro abzuspaltenden Carbonsäurerest im Polymerisat benötigt man 0,05 bis 1,5 Äquivalente an Säure, vorzugsweise 0,4 bis 1,2.

Bei der Hydrolyse mit Basen können Metallhydroxide von Metallen der ersten und zweiten Hauptgruppe des Periodensystems verwendet werden, beispielsweise Lithiumhydroxid, Natriumhydroxid, Kaliumhydroxid, Calciumhydroxid, Strontiumhydroxid und Bariumhydroxid. Ebenso eignen sich aber auch Ammoniak oder Alkylderivate des Ammoniaks, z. B. Alkyl- oder Arylamine wie Triethylamin, Monoethanolamin, Diethanolamin, Triethanolamin, Morpholin, Piperiden, Pyrrolidin oder Anilin. Bei der Hydrolyse mit Basen beträgt der pH-Wert 8 bis 14. Die Basen können in festem, flüssigem oder gegebenenfalls auch gasförmigen Zustand, verdünnt oder unverdünnt eingesetzt werden. Vorzugsweise verwendet man Ammoniak, Natronlauge, oder Kalilauge. Die Hydrolyse im sauren oder alkalischen pH-Bereich erfolgt bei Temperaturen von 20 bis 170°C, vorzugsweise 50 bis 120°C. Sie ist nach etwa 2 bis 8, vorzugsweise 3 bis 5 Stunden beendet.

Besonders bewährt hat sich eine Verfahrensweise, bei der die Säuren oder Basen in wäßriger Lösung zugesetzt werden. Nach der Hydrolyse führt man i.a. eine Neutralisation durch, so daß der pH-Wert der hydrolysierten Polymerlösung 3 bis 12, vorzugsweise 8 bis 11 beträgt. Die Neutralisation ist dann erforderlich, wenn ein Fortschreiten der Hydrolyse von teilweise hydrolysierten Polymerisaten vermieden oder verzögert werden soll.

Die Hydrolyse kann auch mit Hilfe von Enzymen, wie z.B. Amidasen oder Proteasen, vorgenommen werden.

Bei der Hydrolyse tritt gegebenenfalls eine weitere Modifizierung der Polymerisate dahingehend ein, daß die einpolymerisierten Comonomeren ebenfalls hydrolysiert werden. So entstehen beispielsweise aus einpolymerisierten Einheiten von Vinylestern Vinylalkoholeinheiten. In Abhängigkeit von den Hydrolysebedingungnen können die einpolymerisierten Vinylester vollständig oder partiell verseift werden.

Bei einer partiellen Hydrolyse von Vinylacetateinheiten enthaltenden Copolymeren enthält das hydrolysierte Copolymerisat neben unveränderten Vinylacetateinheiten Vinylalkoholeinheiten, sowie N-Vinylcarbonsäureamid- und Vinylamineinheiten. Aus Einheiten monoethylenisch ungesättigter Carbonsäureanhydride, -ester und -amide können bei der Hydrolyse Carbonsäureeinheiten entstehen. Einpolymerisierte monoethylenisch ungesättigte Carbonsäuren selbst werden bei der Hydrolyse nicht verändert. Auch aus einpolymerisierten monoethylenisch ungesättigten Nitrilen können Carbonsäureamid- und Carbonsäureeinheiten gebildet werden. Der Hydrolysegrad der einpolymerisierten Comonomeren kann analytisch leicht bestimmt werden.

Die nach hydrolytischer Spaltung enthaltenden Präpolymerisate haben Molekulargewichte von 1000 bis 10 Millionen, vorzugsweise 10 000 bis 5 Millionen, was K-Werten von etwa 5 bis 300 bzw. 10 bis 250 entspricht, gemessen an 1-%igen wäßrigen Lösungen bei pH 7 und 25°C nach H. Fikentscher, Cellulose-Chemie, Band 13, 58 bis 64 und 71 bis 74 (1932).

Bevorzugt sind Präpolymerisate, die Einheiten von

a) 1 bis 100 mol-% primärem Vinylamin und

b) 0 bis 99 mol-% anderen ethylenisch ungesättigten Monomeren aus der Gruppe N-Vinylformamid, N-Vinylpyrrolidon, N-Vinylcaprolactam, N-Vinylharnstoff, Vinylalkohol, Vinylformiat, Vinylacetat, Vinylpropionat, Acrylsäure und/ oder Acrylnitril enthalten.

Besonders geeignet sind beispielsweise Präpolymerisate, die ausschließlich aus Vinylamineinheiten obiger Formel III bestehen, worin $R^1$ für H, $C_1$-$C_{12}$-Alkyl, $C_3$-$C_{12}$-Cycloalkyl, Aryl, Aralkyl, wobei die Reste $R^1$ sowohl gleich, als auch verschieden sein können. Vorzugsweise geht man von Polymeren mit primären Vinylamin-einheiten ($R^1$ = H) aus.

In Frage kommen als Präpolymerisate auch Copolymerisate, die neben den Vinylamineinheiten bis zu 99,9 mol-%, vorzugsweise bis zu 95 mol-% andere Einheiten ethylenisch ungesättigter Monomere enthalten. Zu nennen sind beispielsweise N-Vinylformamid, N-Vinyl-N-methylformamid, N-Vinyl-N-ethylformamid, N-Vinylpropylformamid, N-Vinyl-N-isopropylformamid, N-Vinyl-N-butylformamid, N-Vinyl-N-sek. butylformamid, N-Vinyl-N-tert.butylformamid, N-Vinyl-N-pentylformamid, Vinylalkohol sowie Vinylester von gesättigten Carbonsäuren mit 1 bis 6 Kohlenstoffatomen,

z.B. Vinylformiat, Vinylacetat, Vinylpropionat und Vinylbutyrat. Geeignet sind auch Copolymere mit ungesättigten $C_3$-$C_8$-Carbonsäuren, wie z. B. Acrylsäure, Methacrylsäure, Maleinsäure, Crotonsäure, Itaconsäure und Vinylessigsäure sowie deren Alkali- und Erdalkalimetallsalzen, Estern, Amiden und Nitrilen, beispielsweise Methylacrylat, Methylmethacrylat, Ethylacrylat, Ethylmethacrylat, Propylacrylat und Butylacrylat oder mit Glykol- bzw. Polyglykolestern ethylenisch ungesättigter Carbonsäuren, wobei jeweils nur eine OH-Gruppe der Glykole und Polyglykole verestert ist, z.B. Hydroxyethylacrylat, Hydroxyethylmethacrylat, Hydroxypropylacrylat, Hydroxypropylmethacrylat, Hydroxybutylacrylat, Hydroxybutylmethacrylat oder auch mit Acrylsäure- und Methacrylsäuremonoestern von Polyalkylenglykolen eines Molgewichts von 1500 bis 10000.

Geeignet als Präpolymerisate sind auch Copolymere, die Ester von ethylenisch ungesättigten Carbonsäuren mit Aminoalkoholen, wie z.B. Dimethylaminoethylacrylat, Dimethylaminoethylmethacrylat, Diethylaminoethylacrylat, Diethylaminoethylmethacrylat, Dimethylaminopropylacrylat, Dimethylaminopropylmethacrylat, Diethylaminopropylacrylat, Diethylaminopropylmethacrylat, Dimethylaminobutylacrylat und Diethylaminobutylacrylat enthalten. Die basischen Acrylate liegen dabei in Form der freien Basen, der Salze mit Mineralsäuren wie z. B. Salzsäure, Schwefelsäure und Salpetersäure, der Salze organischer Säuren, wie Ameisensäure oder Benzolsulfonsäure, oder in quaternisierter Form vor.

Geeignete Quaternisierungsmittel sind beispielsweise Dimethylsulfat, Diethylsulfat, Methylchlorid, Ethylchlorid oder Benzylchlorid.

Außerdem eignen sich als Präpolymerisate Copolymere, die Einheiten ungesättigter Amide, wie beispielsweise Acrylamid, Methacrylamid sowie N-Alkylmono- und -diamide mit Alkylresten von 1 bis 6 Kohlenstoffatomen, z. B. N-Methyl-acrylamid, N,N-Dimethylacrylamid, N-Methylmethacrylamid, N-Ethylacrylamid, N-Propylacrylamid und tert.-Butylacrylamid sowie basische (Meth-)acrylamide, wie z. B. Dimethylaminoethylacrylamid, Dimethylaminoethylmethacrylamid, Diethylaminoethylacrylamid, Diethylaminoethylmethacrylamid, Dimethylaminopropylacrylamid, Dimethylaminopropylmethacrylamid, Diethylaminopropylacrylamid und Diethylaminopropylmethacrylamid, einpolymerisiert enthalten.

Auch Copolymere mit Einheiten von $C_1$-$C_6$-Vinylether, z.B. Vinylmethylether, Vinylethylether, Vinylpropylether, Vinylisopropylether, Vinylbutylether, Vinylisobutylether,Vinylpentylether und Vinylhexylether können als Präpolymerisate eingesetzt werden.

Weiterhin kommen als Präpolymerisate in Frage Copolymere mit Einheiten von N-Vinylpyrrolidon, N-Vinylcaprolactam, N-Vinylharnstoff und substituierten N-Vinylharnstoffen, beispielsweise N-Vinyl-N'-methylharnstoff, N-Vinyl-N'-dimethylharnstoff und N-Vinylimidazol und substituierten N-Vinylimidazolen, wie z. B. N-Vinyl-2-methylimidazol, N-Vinyl-4-methylimidazol, N-Vinyl-5-methylimidazol,N-Vinyl-2-ethylimidazol und N-Vinylimidazolinen wie z.B. N-Vinylimidazolin, N-Vinyl-2-methylimidazolin sowie N-Vinyl-2-ethylimidazolin.

Die Imidazol- und Imidazolinfunktionen liegen dabei in Form der freien Basen oder auch neutralisiert mit Mineralsäuren oder organischen Säuren oder auch quaternisiert vor, wobei die Quaternisierung vorzugsweise mit Dimethylsulfat, Diethylsulfat, Methylchlorid oder Benzylchlorid vorgenommen wird.

Schließlich können als Präpolymerisate auch Copolymere eingesetzt werden, die Monomereinheiten mit Sulfogruppen enthalten, wie beispielsweise Vinylsulfonsäure, Allylsulfonsäure, Methallylsulfonsäure, Styrolsulfonsäure oder Acrylsäure-3-sulfopropylester, Methacrylsäure-3-sulfopropylester und 2-Acrylamido-2-methylpropansulfonsäure. Die Säuregruppen aufweisenden Verbindungen können in Form der freien Säuren, der Ammonium-, Alkalimetall- und Erdalkalimetallsalze eingesetzt werden.

Eine weitere Modifizierung der Präpolymerisate kann dadurch erzielt werden, daß man 0 bis 5 mol-% Einheiten von Monomeren mit mindestens zwei ethylenisch ungesättigten nicht konjugierten Doppelbindungen mit einpolymerisiert. Derartige Comonomere werden üblicherweise bei Copolymerisationen als Vernetzer verwendet. Die Mitverwendung dieser Comonomere während der Copolymerisation bewirkt eine Erhöhung der Molmassen der Copolymerisate. Geeignete Verbindungen dieser Art sind beispielsweise Methylenbisacrylamid, Ester von Acrylsäure und Methacrylsäure mit mehrwertigen Alkoholen, z.B. Glykoldiacrylat, Glycerintriacrylat, Glykoldimethacrylat, Glycerintrimethacrylat sowie mindestens zweifach mit Acrylsäure oder Methacrylsäure veresterte Polyole, wie Pentaerythrit und Glucose. Geeignete Vernetzer sind außerdem Divinylbenzol, Divinyldioxan, Pentaerythrittriallylether, Pentaallylsucrose, Divinylharnstoff und Divinylethylenharnstoff.

Die (Co-)Polymerisation zur Herstellung der Präpolymerisate kann in Gegenwart oder auch in Abwesenheit eines inerten Lösungs- oder Verdünnungsmittels durchgeführt werden. Da die Polymerisation in Abwesenheit von inerten Lösungs- oder Verdünnungsmitteln meistens zu uneinheitlichen Polymerisaten führt, ist die Polymerisation in einem inerten Lösungs- oder Verdünnungsmittel bevorzugt. Geeignet sind beispielsweise solche inerten Lösungsmittel, in denen die offenkettigen N-Vinylcarbonsäureamide löslich sind. Für die Lösungspolymerisation eigenen sich z.B. inerte Lösungsmittel wie Methanol, Ethanol, Isopropanol, n-Propanol, n-Butanol, Tetrahydrofuran, Dioxan, Wasser sowie Mischungen der genannten inerten Lösungsmittel. Die Polymerisation kann kontinuierlich oder diskontinuierlich durchgeführt werden. Sie erfolgt in Gegenwart von radikalbildenden Initiatoren, die in Mengen von 0,01 bis 20, vorzugsweise 0,05 bis 10 Gew.-%, bezogen auf die Monomeren, eingesetzt werden. Gegebenenfalls kann die Polymerisation auch allein durch die Einwirkung von energiereicher Strahlung, z.B. Elektronenstrahlen oder UV-Strahlen initiiert werden.

Um Polymerisate mit niedrigen Molekulargewichten z.B. von 1000 bis 100000, vorzugsweise 5000 bis 50000, herzustellen, wird die Polymerisation zweckmäßigerweise in Gegenwart von Reglern durchgeführt. Geeignete Regler sind beispielsweise organische Verbindungen, die Schwefel in gebundener Form enthalten. Hierzu gehören Mercaptoverbindungen, wie Mercaptoethanol, Mercaptopropanol, Mercaptobutanol, Mercaptoessigsäure, Mercaptopropionsäure, Butylmercaptan, Dodecylmercaptan. Als Regler eignen sich außerdem Allylverbindungen, wie Allylalkohol, Aldehyde, wie Formaldehyd, Acetaldehyd, Propionaldehyd, n-Butyraldehyd und Isobutyraldehyd, Ameisensäure, Ammoniumformiat, Propionsäure, Hydrazinsulfat und Butenole.

Falls die Polymerisation in Gegenwart von Reglern durchgeführt wird, benötigt man davon 0,05 bis 20 Gew.-%, bezogen auf die bei der Polymerisation eingesetzten Monomeren.

Die Polymerisation der Monomeren erfolgt üblicherweise in einer Inertgasatmosphäre unter Ausschluß von Luftsauerstoff. Während der Polymerisation wird im allgemeinen für eine gute Durchmischung der Reaktionsteilnehmer gesorgt. Bei kleineren Ansätzen, bei denen eine sichere Abführung der Polymerisationswärme gewährleistet ist, kann man die Monomeren diskontinuierlich polymerisieren, indem man das Reaktionsgemisch auf die Polymerisationstemperatur erhitzt und dann die Reaktion ablaufen läßt. Diese Temperaturen liegen dabei im Bereich von 40 bis 180°C, wobei unter Normaldruck, vermindertem oder auch erhöhtem Druck gearbeitet werden kann. Polymerisate mit einem hohen Molekulargewicht erhält man, wenn man die Polymerisation in Wasser durchführt. Dies kann beispielsweise zur Herstellung wasserlöslicher Polymerisate in wäßriger Lösung, als Wasser-in-Öl-Emulsion oder nach dem Verfahren der umgekehrten Suspensionspolymerisation erfolgen.

Um eine Verseifung der monomeren N-Vinylcarbonsäureamide während der Polymerisation in wäßriger Lösung zu vermeiden, führt man die Polymerisation vorzugsweise in einem pH-Bereich von 4 bis 9, insbesondere von 5 bis 8 durch. In vielen Fällen empfiehlt es sich, zusätzlich noch in Gegenwart von Puffern zu arbeiten, z. B. primärem oder sekundärem Natriumphosphat.

Die erfindungsgemäßen Polymerisate, insbesondere solche mit Monomer-Einheiten der Formeln I und/oder II, finden Anwendung als Wirkstoffe in kosmetischen Zubereitungen, beispielsweise als Conditioner für Shampoos, Haarkuren, Lotionen, Emulsionen, Spülungen, Gele, Schäume und Vor- und Nachbehandlungsmittel für Haarfärbung und Dauerwelle sowie als Haarfestiger und Haarstylingmittel mit Pflegeeigenschaften. Weiterhin können sie als Verdicker in Kosmetikformulierungen und in kosmetischen Zubereitungen für Mundpflege eingesetzt werden. Überraschenderweise wurde gefunden, daß die erfindungsgemäßen N-Vinylcarbonsäureamidcopolymere verbesserte Eigenschaften in kosmetischen Formulierungen aufweisen. Am auffälligsten sind diese Eigenschaftsverbesserungen in Shampooformulierungen. Bevorzugt verwendet man die Polymerisate in marktüblichen Shampooformulierungen mit Natrium-bzw. Ammoniumlaurylethersulfat als Basistensid und gegebenenfalls weiteren Cotensiden, z.B. Alkylpolyglycoside, Cocamidopropylbetaine, Sulfobernsteinsäureester, sek.-Alkansulfonate, α-Olefinsulfonate, Eiweißfettsäurekondensate, N-Acylsarkosinate, Tauride, Methyltauride, Fettsäureisothionate, N-Acylglutamate, Ethercarbonsäurederivate, Alkylphosphatester, Alkylphosphatester, Alkylbetaine, Alkylamidopropylbetaine, Sulfobetaine, Alkylglycerylethersulfonate, Cocosamphocarboxyglycinate und Sobitanderivate. Nach Anwendung der erfindungsgemäßen Polymere in den oben genannten Shampoos wird insbesondere die Naßkämmbarkeit des Haares verbessert. Zudem werden dem Haar Glanz, Volumen und Body sowie hervorragende Festigkeit und antistatische Eigenschaften verliehen.

Die Shampoos haben somit Wasch-, Konditionier- und Festigerwirkung (3 in 1-Shampoos), wobei die Polymere schon in niedrigen Einsatzmengen zur Wirkung kommen. Gute Konditionier- und Festigungseffekte zeigen die N-Vinylamineinheiten enthaltenden Copolymere auch in Haarkuren, Lotionen, Emulsionen, Spülungen sowie Stylingmitteln, wie Gelen und Schäumen, und in Vor- und Nachbehandlungsmitteln für Haarfärbung und Dauerwelle, wobei sie hervorragende Pflegeeigenschaften bewirken.

Weiterhin können die Copolymere auch als Konditioniermittel und Verdicker in Hautpflegemitteln, wie z.B. in Cremen, Salben, Emulsionen und Lotionen, sowie für die Mundpflege, wie z.B. in Zahnpasten, Gelen und Mundwässern, eingesetzt werden.

Die erfindungsgemäßen Polymerisate sind in den kosmetischen Mitteln gewöhnlich in einem Anteil von etwa 0,01 bis 15 Gew.-%, wie z.B. etwa 0,1 bis 10 Gew.-%, bezogen auf das Gesamtgewicht des Mittels, enthalten. Neben üblichen kosmetischen Trägern können weitere übliche Zusätze, wie z.B. oberflächenaktive Mittel, Verdickungsmittel, gelbildende Mittel, Lösungsvermittler, Feuchthaltemittel, Bindemittel, Treibmittel, Polymere, wie z.B. Silikone, Sequestriermittel, Chelatbildner, Viskositätsmodifikatoren, Trübungsmittel, Stabilisatoren, Perlglanzmittel, Farbstoffe, Duftstoffe, organische Lösungsmittel, Konservierungsmittel, pH-einstellende Mittel, und gegebenenfalls weitere Konditioniermittel enthalten sein.

Die Erfindung wird nun anhand der folgenden nichtlimitierenden Beispiele näher erläutert.

Herstellungsbeispiele

In den folgenden Beispielen 1 bis 9 wird die Herstellung einiger erfindungsgemäßer Polymerisate beschrieben.

Beispiel 1

In einer Rührapparatur mit Rückflußkühler, Thermometer und Tropftrichter werden 500 g einer wäßrigen Lösung von Polyvinylamin, Polymergehalt 6,6 Gew.-% (≅ 767 mmol Vinylamineinheiten), pH-Wert 10, K-Wert 112 ($M_w$ ≅ 300000) vorgelegt. Unter kräftigem Rühren werden im Verlauf von 10 min. 30 g (≅ 348 mmol) Butyrolacton zugetropft. Daraufhin erhitzt man das Reaktionsgemisch 3 h auf 80°C. Nach Abkühlung auf Raumtemperatur wird die Lösung mit 7,5 g konzentrierter Ameisensäure auf einen pH-Wert von 7 eingestellt. Man erhält 537,5 g Copolymerlösung mit einer Viskosität von 13440 mPas (Brookfield, 20°C). Das resultierende Polymer setzt sich aus 66,5 mol-% Vinylamin- und 33,5 mol-% N-Vinyl-γ-hydroxybuttersäureamideinheiten zusammen. Demnach wurden 74 % des eingesetzten Butyrolactons umgesetzt. Der Polymergehalt beträgt 9,4 Gew.-%.

Beispiel 2

Die Umsetzung erfolgt wie in Beispiel 1 beschrieben mit 500 g Polyvinylaminlösung, Polymergehalt 6,6 Gew.-% (≅ 767 mmol Vinylamineinheiten), pH-Wert 10, K-Wert 112 ($M_w$ ≅ 300000), 19,8 g (≅ 230 mmol) Butyrolacton und 4,6 g Ameisensäure. Man erhält 524,4 g Copolymerlösung mit einem einem Polymergehalt von 8,6 % und einer Brookfield-Viskosität von 11700 mPas (Brookfield, 20°C). Der Umsatz (bzgl. Butyrolacton) beträgt 72 %. Das Copolymer ist aus 78,4 mol-% Vinylamin und 21,6 mol-% N-Vinyl-γ-hydroxybuttersäureamid zusammengesetzt.

Beispiel 3

Die Umsetzung erfolgt wie in Beispiel 1 beschrieben mit 500 g Polyvinylaminlösung, 38,0 g (≅ 441 mmol) Butyrolacton und 0,7 g Ameisensäure. Man erhält 538,7 g Copolymerlösung mit einem Polymergehalt von 12,9 % und einer Brookfield-Viskosität von 15180 mPas. Der Umsatz (bzgl. Butyrolacton) beträgt 83,6 %. Das Copolymer ist aus 58,2 mol-% Vinylamin und 41,8 mol-% N-Vinyl-γ-hydroxybuttersäureamid zusammengesetzt.

Beispiel 4

In einer Rührapparatur mit Rückflußkühler, Thermometer und Tropftrichter werden 500 g einer wäßrigen Lösung eines Copolymeren aus 70 mol-% Polyvinylamin und 30 mol-% N-Vinylformamid, Polymergehalt 7,7 Gew.-%, (≅ 524 mmol Vinylamineinheiten), pH-Wert 10, K-Wert 88, vorgelegt. Unter kräftigem Rühren werden im Verlauf von 10 min. 13,5 g (≅ 156,8 mmol) Butyrolacton zugetropft. Daraufhin erhitzt man das Reaktionsgemisch 5 h lang auf 70°C. Nach Abkühlung auf Raumtemperatur wird die Lösung mit 14 g konzentrierter Salzsäure auf einen pH-Wert von 5 eingestellt. Man erhält 527,5 g Copolymerlösung mit einer Viskosität von 13250 mPas (Brookfield, 20°C). Das resultierende Polymer setzt sich aus 56 mol-% Vinylamin-, 30 mol-% N-Vinylformamid- und 14 mol-% N-Vinyl-γ-hydroxybuttersäureamideinheiten zusammen. Demnach wurden 67,3 % des eingesetzten Butyrolactons umgesetzt. Der Polymergehalt beträgt 9,0 Gew.-%.

Beispiel 5

Die Umsetzung erfolgt wie in Beispiel 4 beschrieben mit 500 g Copolymerlösung, 22,6 g (≅ 262 mmol) Butyrolacton und 8 g Salzsäure. Man erhält 530,6 g Copolymerlösung mit einem Polymergehalt von 10,2 Gew.-% und einer Brookfield-Viskosität von 16105 mPas. Der Umsatz (bzgl. Butyrolacton) beträgt 69,8 %. Das Copolymer ist aus 46 mol-% Vinylamin-, 30 mol-% N-Vinylformamid- und 24 mol-% N-Vinyl-γ-hydroxy-buttersäureamideinheiten zusammengesetzt.

Beispiel 6

In einer Rührapparatur mit Rückflußkühler, Thermometer und Tropftrichter werden 500 g einer wäßrigen Lösung von Polyvinylamin, Polymergehalt 22,6 Gew.-%, (≅ 2616 mmol Vinylamineinheiten), pH-Wert 9, K-Wert 30 ($M_w$ ≅ 10-20000), vorgelegt. Unter kräftigem Rühren werden im Verlauf von 20 min. 89,4 g (≅ 783 mmol) Caprolacton zugetropft. Daraufhin erhitzt man das Reaktionsgemisch 5 h lang auf 80°C. Man erhält 589,4 g Copolymerlösung. Das resultierende Polymer setzt sich aus 73,5 mol-% Vinylamin- und 26,5 mol-% N-Vinyl-6-hydroxycapronsäureamideinheiten zusammen. Demnach wurden 88,4 % des eingesetzten Caprolactons umgesetzt. Der Polymergehalt beträgt 32,5 Gew.-%.

Beispiel 7

Die Umsetzung erfolgt wie in Beispiel 6 beschrieben mit 500 g Copolymerlösung, 149,3 g (≅ 1308 mmol) Caprolacton. Man erhält 649,3 g Copolymerlösung mit einem Polymergehalt von 35,0 Gew.-%. Der Umsatz (bzgl. Caprolacton) beträgt 76,9 %.

Das Copolymer ist aus 61,5 mol-% Vinylamin- und 38,5 mol-% N-Vinyl-6-hydroxy-capronsäureamideinheiten zusammengesetzt.

Beispiel 8

Die Umsetzung erfolgt wie in Beispiel 6 beschrieben mit 500 g Copolymerlösung, 209 g (≅ 1831 mmol) Caprolacton. Man erhält 709 g Copolymerlösung mit einem Polymergehalt von 34,7 Gew.-%. Der Umsatz (bzgl. Caprolacton) beträgt 63,7 %.

Das Copolymer ist aus 55,4 mol-% Vinylamin- und 44,6 mol-% N-Vinyl-6-hydroxycapronsäureamideinheiten zusammengesetzt.

Beispiel 9

In einer Rührapparatur mit Rückflußkühler, Thermometer und Tropftrichter werden 500 g einer wäßrigen Lösung von Polyvinylamin, Polymergehalt 25,2 Gew.-% (≅ 2927 mmol Vinylamineinheiten), pH-Wert 10, K-Wert 30 ($M_w$ = 10-20000) vorgelegt. Unter kräftigem Rühren werden im Verlauf von 30 min. 280 g (≅ 2057 mmol) Benzoesäuremethylester zugetropft. Man fügt 300 g N-Methylpyrrolidon dazu und erhitzt das Reaktionsgemisch 8 h auf 90°C. Nach Abkühlung auf Raumtemperatur wird die Lösung mit 7,5 g konzentrierter Ameisensäure auf einen pH-Wert von 7 eingestellt. Man erhält 1087,5 g Copolymerlösung. Das resultierende Polymer setzt sich aus 57,8 mol-% Vinylamin- und 42,2 mol-% N-Vinylbenzamideinheiten zusammen. Demnach wurden 60% des eingesetzten Benzoesäuremethylesters zu Amidfunktionen umgesetzt. Der Polymergehalt beträgt 23,4 Gew.-%.

Anwendungstechnische Beispiele

Beispiel 10: Conditioner-Wirkung erfindungsgemäßer Polymerisate

Die erfindungsgemäßen Polymere gemäß Beispiel 1 und 2 (Polymer 1 bzw. 2) wurden mit den bekannten Conditioner-Polymeren 3 und 4 bezüglich ihrer Conditionerwirkung auf Kopfhaar verglichen.

Polymer 1: Copolymer aus 66,5 mol-% Vinylamin und 33,5 mol-% N-Vinyl-γ-hydroxybuttersäureamid mit einem Molekulargewicht von ca. 500 000.

Polymer 2: Copolymer aus 78,4 mol-% Vinylamin und 21,6 mol-% N-Vinyl-γ-hydroxybuttersäureamid mit einem Molekulargewicht von ca. 500 000.

Polymer 3 (Vergleich): Handelsübliches Copolymerisat aus Acrylamid und Diallyldimethylammoniumchlorid mit einem mittleren Molekulargewicht von ca. 1 000 000 (Merquat® S von Merck Calgon).

Polymer 4 (Vergleich): Handelsübliche kationische Hydroxyethylcellulose (Celquat® H 100 von National Starch)

Zur Prüfung als Conditioniermittel für Haarshampoos wurden die genannten Polymere 1 bis 4 in angegebener Menge einem Standard-Testshampoo mit 15,0 Gew.-% Natriumlaurylethersulfat mit 2 bis 3 Ethylenoxideinheiten (Texapon® NSO von Henkel KG) zugesetzt. Anhand von definierten Haartressen wurde der Einfluß der Polymeren auf die Naßkämmbarkeit getestet. Dazu wurden die Haartressen mit dem polymerhaltigen Testshampoo gewaschen und gespült und dann die Kämmkraft gemessen. Als Blindprobe diente eine Haartresse, die mit Testshampoo ohne Zusatz behandelt wurde. Die Ergebnisse sind in Tabelle 1 aufgeführt.

Die Kämmkraftabnahme errechnet sich nach folgender Formel:

$$\text{Abnahme [\%]} = \frac{\text{Meßwert x 100}}{\text{Blindwert}} - 100$$

Demnach sind hohe Kämmkraftabnahmen als positiv zu bewerten.

Tabelle 1:

| Polymer | Zusatzmenge (Gew.-%) | Kämmkraftabnahme (% bezüglich Blindwert) |
|---|---|---|
| 1 | 0,1 | 38 |
| 2 | 0,1 | 30 |
| 3 (Vergleichsbeispiel) | 1,0 | 18 |
| 4 (Vergleichsbeispiel) | 0,1 | 25 |

58/hz

## Patentansprüche

1. Polymerisat, **dadurch gekennzeichnet, daß** es als wesentliche Strukturelemente Einheiten der Formel I und/oder II

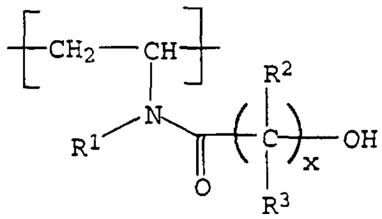

(I)

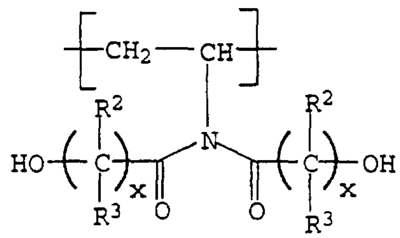

(II)

umfaßt, worin

$R^1$ für H, Alkyl, Cycloalkyl, Aryl oder Aralkyl steht,
$R^2$ und $R^3$ unabhängig voneinander wie $R^1$ definiert sind oder ausgewählt sind unter substituiertem Alkyl, substituiertem Cycloalkyl, substituiertem Aryl oder substituiertem Aralkyl, wobei die Substituenten ausgewählt sind unter OH, O-Alkyl, O-Aryl, SH, S-Alkyl, S-Aryl, $NH_2$, NH-Alkyl, NH-Aryl, $N(Alkyl)_2$, gegebenenfalls in protonierter Form, $N(Alkyl)_3^+Z^-$, worin Z für den Rest einer anorganischen oder organischen Säure steht, COOH, COO-Alkyl, $CONH_2$, CONH-Alkyl, $CON(Alkyl)_2$, CN und $SO_3H$; und

die Indices x jeweils unabhängig voneinander für eine ganze Zahl von 1 bis 20 stehen;
und die entsprechenden Säureadditionssalze davon.

2. Polymerisat nach Anspruch 1, **dadurch gekennzeichnet, daß** es ein gewichtsmittleres Molekulargewicht von etwa $10^3$ bis etwa $10^8$ aufweist.

3. Polymerisat nach einem der vorhergehenden Ansprüche, **dadurch gekennzeichnet, daß** es

a) 0,1 bis 100 mol-% Einheiten obiger allgemeiner Formeln I und/oder II
b) 0 bis 99,9 mol-% Vinylamineinheiten der allgemeinen Formel III

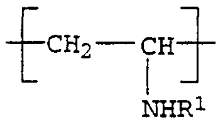 (III)

worin $R^1$ die oben angegebenen Bedeutungen besitzt,
c) 0 bis 99,9 mol-% von a) und b) verschiedener monoethylenisch ungesättigter Monomereinheiten; und
d) 0 bis 5 mol-% Einheiten von Monomeren mit mindestens zwei ethylenisch ungesättigten Doppelbindungen;

umfaßt.

**4.** Verfahren zur Herstellung eines Polymerisats, das als wesentliche Strukturelemente Einheiten der Formel IV und/oder V

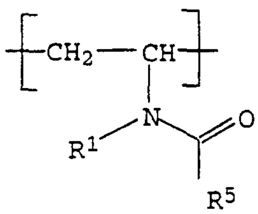 (IV) 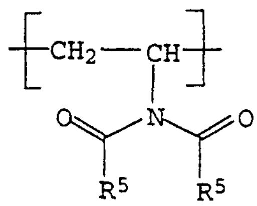 (V)

umfaßt, worin

$R^1$ die oben angegebenen Bedeutungen besitzt, und
die Reste $R^5$ unabhängig voneinander ausgewählt sind unter Alkyl, Cycloalkyl, Aryl und Aralkyl, gegebenenfalls ein- oder mehrfach substituiert mit OH, 0-Alkyl, O-Aryl, SH, S-Alkyl, S-Aryl, $NH_2$, NH-Alkyl, NH-Aryl, $N(Alkyl)_2$, gegebenenfalls in protonierter Form, $N(Alkyl)_3^+Z^-$, worin Z für den Rest einer anorganischen oder organischen Säure steht, COOH, COO-Alkyl, $CONH_2$, CONH-Alkyl, $CON(Alkyl)_2$, CN und $SO_3H$; oder für einen Rest der Formel VI

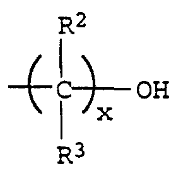 (VI)

stehen, worin $R^2$, $R^3$ und x die oben angegebenen Bedeutungen besitzen und $R^2$ und $R^3$ außerdem unabhängig voneinander für OH stehen können,

**dadurch gekennzeichnet, daß** man ein Präpolymerisat, enthaltend Vinylamineinheiten der Formel III

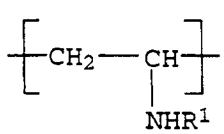 (III)

worin $R^1$ die oben angegebenen Bedeutungen besitzt, mit einer Verbindung der Formel VII

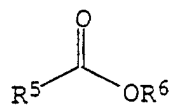

(VII)

worin

$R^5$ die oben angegebenen Bedeutungen besitzt und
$R^6$ für Alkyl, Aralkyl oder Aryl steht, oder
$R^5$ und $R^6$ zusammen für eine Gruppe der Formel VIa

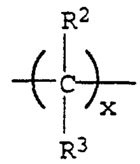

(VIa)

stehen, worin $R^2$, $R^3$ und x die oben angegebenen Bedeutungen besitzen,

umsetzt.

**5.** Verfahren nach Anspruch 4, **dadurch gekennzeichnet, daß** man das Präpolymerisat mit einer Verbindung der Formel VIII

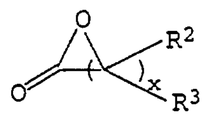

(VIII)

worin
$R^2$, $R^3$ und x die oben angegebenen Bedeutungen besitzen, und $R^2$ und $R^3$ außerdem unabhängig voneinander für OH stehen können, umsetzt.

**6.** Verfahren nach Anspruch 4 oder 5, **dadurch gekennzeichnet, daß** man ein Präpolymerisat einsetzt, das

b) 0,1 bis 100 mol-% Vinylamineinheiten der Formel III,
c) 0 bis 99,9 mol-% von a) verschiedener monoethylenisch ungesättigter Monomereinheiten und
d) 0 bis 5 mol-% Einheiten von Monomeren mit mindestens zwei ethylenisch ungesättigten Doppelbindungen umfaßt.

**7.** Verfahren nach Anspruch 4, 5 oder 6, **dadurch gekennzeichnet, daß** die Umsetzung in Gegenwart von Wasser erfolgt.

**8.** Verwendung eines Polymerisats gemäß einem der Ansprüche 1 bis 3, wobei $R^2$ und $R^3$ außerdem unabhängig voneinander für OH stehen können, in kosmetischen Mitteln.

**9.** Verwendung nach Anspruch 8 als Konditionier- oder Verdickungsmittel.

**10.** Verwendung nach Anspruch 8 oder 9, **dadurch gekennzeichnet, daß** das kosmetische Mittel ausgewählt ist unter haarkosmetischen Mitteln, Hautpflegemitteln und Mundpflegemitteln.

**11.** Verwendung nach Anspruch 9 oder 10 in Haarfestigern, Haarkuren, Lotionen, Emulsionen, Spülungen, Gelen, Schäumen, Vor- und Nachbehandlungsmitteln für Haarfärbung und Dauerwelle, Haarstylingmitteln mit Pflegeeigenschaften, oder Shampoos.

**12.** Verwendung nach Anspruch 11 als Konditioniermittel für Shampoos.

**13.** Kosmetisches Mittel, enthaltend wenigstens ein Polymerisat gemäß einem der Ansprüche 1 bis 3, wobei $R^2$ und $R^3$ außerdem unabhängig voneinander für OH stehen können, in einem kosmetischen Träger; gegebenenfalls in Kombination mit weiteren kosmetisch aktiven Substanzen.

## Claims

**1.** A polymer whose essential structural elements comprise units of the formula I and/or II

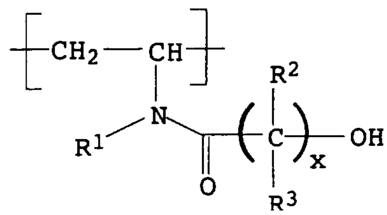

(I)

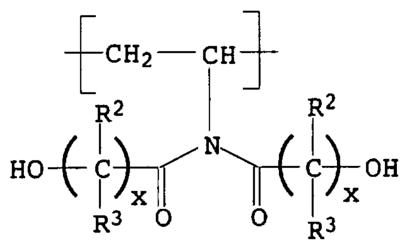

(II)

where

| | |
|---|---|
| $R^1$ | is H, alkyl, cycloalkyl, aryl or aralkyl, |
| $R^2$ and $R^3$ | independently are as defined for $R^1$ or are selected from substituted alkyl, substituted cycloalkyl, substituted aryl or substituted aralkyl, the substituents being selected from OH, O-alkyl, O-aryl, SH, S-alkyl, S-aryl, $NH_2$, NH-alkyl, NH-aryl, $N(alkyl)_2$, possibly in protonated form, $N(alkyl)_3^+Z^-$, where Z is the radical of an inorganic or organic acid, COOH, COO-alkyl, $CONH_2$, CONH-alkyl, $CON(alkyl)_2$, CN and $SO_3H$; and |

the indices x are each independently an integer from 1 to 20;
or a corresponding acid addition salt thereof.

**2.** A polymer as claimed in claim 1, which has a weight-average molecular weight of from about $10^3$ to about $10^8$.

**3.** A polymer as claimed in either of the preceding claims, which comprises

a) from 0.1 to 100 mol- % of units of the above formulae I and/or II,
b) from 0 to 99.9 mol-% of vinylamine units of the formula III

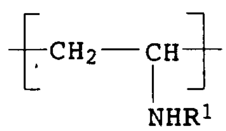

(III)

where $R^1$ is as defined above,
c) from 0 to 99.9 mol-% of monoethylenically unsaturated monomer units other than a) and b); and
d) from 0 to 5 mol-% of units of monomers having at least two ethylenically unsaturated double bonds.

**4.** A process for preparing a polymer whose essential structural elements comprise units of the formula IV and/or V

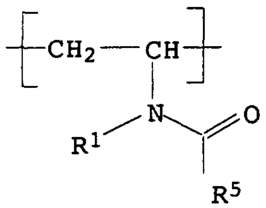 (IV) 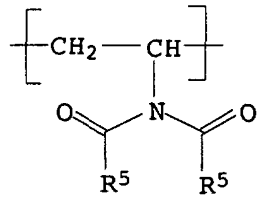 (V)

where

| | |
|---|---|
| $R^1$ | is as defined above and |
| the radicals $R^5$ | independently are selected from alkyl, cycloalkyl, aryl and aralkyl, unsubstituted or mono- or polysubstituted by OH, O-alkyl, O-aryl, SH, S-alkyl, S-aryl, $NH_2$, NH-alkyl, NH-aryl, $N(alkyl)_2$, possibly in protonated form, $N(alkyl)_3^+Z^-$, where Z is the radical of an inorganic or organic acid, COOH, COO-alkyl, $CONH_2$, CONH-alkyl, $CON(alkyl)_2$, CN and $SO_3H$; or are a radical of the formula VI |

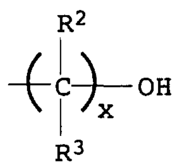 (VI)

where $R^2$, $R^3$ and x are as defined above and $R^2$ and $R^3$, additionally and independently, can be OH,

which comprises reacting a prepolymer comprising vinylamine units of the formula III

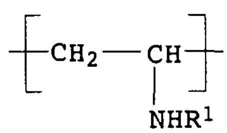 (III)

where $R^1$ is as defined above, with a compound of the formula VII

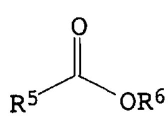 (VII)

where

| | |
|---|---|
| $R^5$ | is as defined above and |
| $R^6$ | is alkyl, aralkyl or aryl, or |
| $R^5$ and $R^6$ | together are a group of the formula VIa |

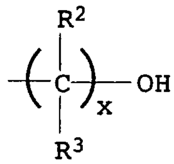

(VIa)

where $R^2$, $R^3$ and x are as defined above.

**5.** A process as claimed in claim 4, wherein the prepolymer is reacted with a compound of the formula VIII

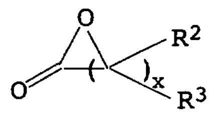

(VIII)

where
$R^2$, $R^3$ and x are as defined above and $R^2$ and $R^3$, additionally and independently, can be OH.

**6.** A process as claimed in claim 4 or 5, wherein a prepolymer is used which comprises

b) from 0.1 to 100 mol-% of vinylamine units of the formula III,
c) from 0 to 99.9 mol-% of monoethylenically unsaturated monomer units other than a) and b); and
d) from 0 to 5 mol-% of units of monomers having at least two ethylenically unsaturated double bonds.

**7.** A process as claimed in claim 4, 5 or 6, wherein the reaction takes place in the presence of water.

**8.** The use of a polymer as claimed in any of claims 1 to 3, where $R^2$ and $R^3$, additionally and independently, can be OH, in cosmetic compositions.

**9.** The use as claimed in claim 8 as a conditioning or thickening composition.

**10.** The use as claimed in claim 8 or 9, wherein the cosmetic composition is selected from hair cosmetics compositions, skin-care compositions and oral care compositions.

**11.** The use as claimed in claim 9 or 10 in hairsetting compositions, hair remedies, lotions, emulsions, rinses, gels, mousses, pretreatment and aftertreatment compositions for hair coloring and permanent waving, hairstyling compositions having care properties, or shampoos.

**12.** The use as claimed in claim 11 as conditioning agent for shampoos.

**13.** A cosmetic composition comprising at least one polymer as claimed in any of claims 1 to 3, in which $R^2$ and $R^3$, additionally and independently, can be OH, in a cosmetic vehicle, alone or in combination with further cosmetically active substances.

**Revendications**

**1.** Polymère, **caractérisé par le fait qu'**il comporte comme éléments structuraux essentiels des unités de formule I et/ou II

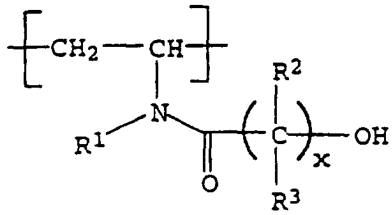

(I)

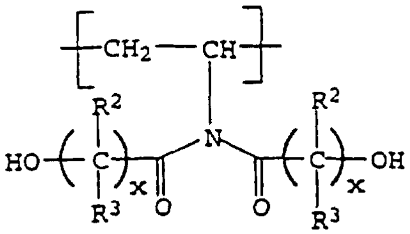

(II)

où

| | |
|---|---|
| $R^1$ | désigne H, alkyle, cycloalkyle, aryle ou aralkyle, |
| $R^2$ et $R^3$ | sont, indépendamment l'un de l'autre, définis comme $R^1$ ou choisis parmi les groupes alkyle substitué, cycloalkyle substitué, aryle substitué ou aralkyle substitué, tandis que les substituants sont choisis parmi OH, O-alkyle, O-aryle, SH, S-alkyle, S-aryle, $NH_2$,NH-alkyle, NH-aryle, N (alkyle)$_2$, éventuellement sous forme protonée, N(alkyle)$_3{}^+Z^-$, où Z représente le reste d'un acide inorganique ou organique, COOH, COO-alkyle, $CONH_2$, CONH-alkyle, CON(alkyle)$_2$, CN et $SO_3H$; et |
| les indices x | représentent, à chaque fois indépendamment l'un de l'autre, un nombre entier de 1 à 20; |

et leurs sels correspondants d'addition à un acide.

**2.** Polymère selon la revendication 1, **caractérisé par le fait qu'**il présente une masse moléculaire moyenne en poids d'environ $10^3$ à environ $10^8$.

**3.** Polymère selon l'une des revendications précédentes, **caractérisé par le fait qu'**il comporte

a) 0,1 à 100% en mol d'unités de formules générales I et/ou II susdites
b) 0 à 99,9% en mol d'unités vinylamine de formule générale III

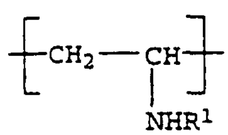

(III)

où $R^1$ possède les significations indiquées plus haut,
c) 0 à 99,9% en mol d'unités monomères à insaturation monoéthylénique différentes de a) et b); et
d) 0 à 5% en mol d'unités de monomères ayant au moins deux double liaisons à insaturation éthylénique.

**4.** Procédé pour la préparation d'un polymère, qui comporte comme éléments structuraux essentiels des unités de formule IV et/ou V

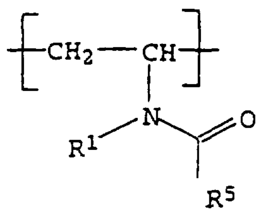 (IV) 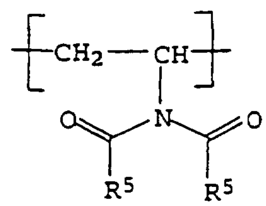 (V)

où

- $R^1$ possède les significations indiquées plus haut, et
- les restes $R^5$ sont, indépendamment l'un de l'autre, choisis parmi alkyle, cycloalkyle, aryle et aralkyle, éventuellement substitué une ou plusieurs fois par OH, O-alkyle, O-aryle, SH, S-alkyle, S-aryle, $NH_2$, NH-alkyle, NH-aryle, $N(alkyle)_2$, éventuellement sous forme protonée, $N(alkyle)_3{}^+Z^-$, où Z représente le reste d'un acide inorganique ou organique, COOH, COO-alkyle, $CONH_2$, CONH-alkyle, $CON(alkyle)_2$, CN et $SO_3H$; ou un reste de formule VI

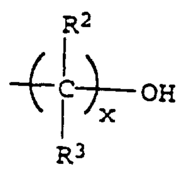 (VI)

où $R^2$, $R^3$ et x possèdent les significations indiquées plus haut, et $R^2$ et $R^3$ peuvent en outre désigner OH, indépendamment l'un de l'autre,

**caractérisé par le fait qu'**on fait réagir un prépolymère, contenant des unités vinylamine de formule III

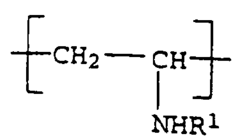 (III)

où $R^1$ possède les significations indiquées plus haut, avec un composé de formule VII

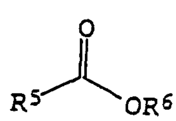 (VII)

où

- $R^5$ possède les significations indiquées plus haut et
- $R^6$ représente alkyle, aralkyle ou aryle, ou
- $R^5$ et $R^6$ représentent ensemble un groupe de formule VIa

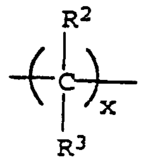

(VIa)

où $R^2$, $R^3$ et x possèdent les significations indiquées plus haut.

**5.** Procédé selon la revendication 4, **caractérisé par le fait qu'**on fait réagir le prépolymère avec un composé de formule VIII

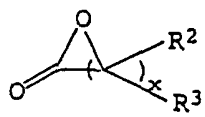

(VIII)

où
$R^2$, $R^3$ et x possèdent les significations indiquées plus haut, et $R^2$ et $R^3$ peuvent en outre, indépendamment l'un de l'autre, représenter OH.

**6.** Procédé selon la revendication 4 ou 5, **caractérisé par le fait qu'**on utilise un prépolymère qui comporte

b) 0,1 à 100% en mol d'unités vinylamine de formule III,
c) 0 à 99,9% en mol d'unités monomères à insaturation monoéthylénique différentes de a) et
d) 0 à 5% en mol d'unités de monomères ayant au moins deux liaisons doubles à insaturation éthylénique.

**7.** Procédé selon la revendication 4, 5 ou 6, **caractérisé par le fait que** la réaction s'effectue en présence d'eau.

**8.** Utilisation d'un polymère selon l'une des revendications 1 à 3, dans laquelle $R^2$ et $R^3$ peuvent en outre, indépendamment l'un de l'autre, représenter OH, dans des produits cosmétiques.

**9.** Utilisation selon la revendication 8 comme agent de conditionnement ou d'épaississement.

**10.** Utilisation selon la revendication 8 ou 9, **caractérisée par le fait que** le produit cosmétique est choisi parmi des produits cosmétiques pour les cheveux, des produits de soins pour la peau et des produits de soins buccaux.

**11.** Utilisation selon la revendication 9 ou 10 dans des renforçateurs pour cheveux, des traitements pour cheveux, des lotions, des émulsions, des produits de lavage, des gels, des mousses, des produits de pré- et de post-traitement pour teinture des cheveux et mise en plis, des produits de style pour cheveux ayant des propriétés d'entretien, ou des shampooings.

**12.** Utilisation selon la revendication 11 comme produit de conditionnement pour shampooings.

**13.** Produit cosmétique, contenant au moins un polymère selon l'une des revendications 1 à 3, dans lequel $R^2$ et $R^3$ peuvent en outre, indépendamment l'un de l'autre, représenter OH, dans un support cosmétique, éventuellement en combinaison avec d'autres substances à activité cosmétique.